# FASCICULE DE BREVET EUROPEEN

(11) **EP 0 930 059 B1**
(45) Date de publication et mention de la délivrance du brevet: **22.05.2002**
(21) Numéro de dépôt: 98403262.3
(22) Date de dépôt: 22.12.1998
(51) Int. Cl.: A61K 7/00, A61K 7/42, A61K 7/48

(54) **Utilisation d'un solvant polyfluoré volatil, en tant qu'agent accélérateur de sèchage, dans des produits cosmétiques**
Verwendung von einem flüchtigen Polyfluor-enthaltenden Lösungsmittel als Trocknungsbeschleuniger in kosmetischen Produkten
Use of a volatile polyfluorinated solvent as a drying accelerator in cosmetical products

(30) Priorité: 29.12.1997 FR 9716628; 30.06.1998 FR 9808338
(43) Date de publication de la demande: 21.07.1999
(73) Titulaire: L'OREAL, 75008 Paris (FR)
(72) Inventeur: Bara, Isabelle, 75013 Paris (FR)
(74) Mandataire: Larcher, Dominique

(56) Documents cités:
- EP-A- 0 558 423
- EP-A- 0 595 683
- FR-A- 2 593 392
- FR-A- 2 756 176
- BASE DE DONN ES "CHEMICAL ABSTRACTS" (SERVEUR: STN): Abr.117:137 461, Colombus, OH, USA; & JP 04 139 121 A (TOSHIBA SILICONE CO.) 13 MAI 1992 XP002077150
- BASE DE DONN ES "CHEMICAL ABSTRACTS" (SERVEUR: STN): Abr.110: 160 222, Colombus, OH, USA; & JP 63 002 916 A (KANEBO, Ltd) 7 JANVIER 1988 XP002077151
- BASE DE DONN ES "CHEMICAL ABSTRACTS" (SERVEUR: STN): Abr.107:161 389, Colombus, OH, USA; & JP 62 093 211 A (SHISEIDO CO., Ltd) 28 AVRIL 1987 XP002086747

## Description

La présente invention a pour objet l'utilisation d'une certaine classe de composés polyhalogéno organiques volatils, dont l'atome d'halogène est le fluor, dans des compositions cosmétiques de maquillage, de protection solaire ou de soin des ongles en vue d'en accélérer le temps de séchage.

Le problème lié au séchage des produits cosmétiques après leur application revêt une grande importance pour les utilisatrices.

En effet, un produit ayant une vitesse de séchage lente est généralement mal accepté, qu'il s'agisse d'un fond de teint, d'un rouge à lèvres ou d'un produit pour le soin des ongles.

Par ailleurs, la vitesse de séchage ne doit pas non plus être trop rapide car elle serait alors susceptible de produire des phénomènes de retrait inesthétiques.

Bien que diverses études aient été réalisées en vue d'améliorer le temps de séchage des compositions cosmétiques, notamment de maquillage, aucune solution satisfaisante n'a cependant été proposée.

Dans la demande EP 558 423, il a été décrit des dispersions huile-dans-l'eau susceptibles de former des films composites sur divers substrats kératiniques comprenant une phase fluorée constituée d'au moins un composé organofluoré hydrocarboné. Toutefois dans cette demande, le composé organofluoré hydrocarboné n'est pas du type volatil dans la mesure où il est présent dans le film composite conduisant à un toucher plus doux et plus agréable du film.

On doit également citer la demande EP 1 029 527 qui propose, pour remédier aux effets néfastes des composés chlorofluoro-carbonés, l'utilisation, dans des compositions cosmétiques, de composés fluorés, en particulier d'hydrofluroéthers, ceux-ci permettant par ailleurs une évaporation rapide et la formation d'un revêtement uniforme maintenant l'humidité de la peau.

Les compositions de cette demande sont décrites comme se présentant sous forme de fond de teint, de poudre pour le visage, de fard à joues, de fard à paupières, de lotion pour le visage, de lotion laiteuse, de crème et de lotion laiteuse anti-solaire, de produits pour les soins des cheveux tels que des produits de rinçage et de mise en forme ainsi sous forme de brillants à lèvres.

Après de nombreuses études sur divers types de composés, on a constaté de façon surprenante et inattendue, qu'en utilisant une certaine classe de solvants polyhalogéno organiques volatils, dont l'atome d'halogène est le fluor, il était possible d'améliorer, d'une façon particulièrement satisfaisante et efficace, le temps de séchage des compositions cosmétiques.

En effet, l'utilisation de ces solvants, ci-après désignés par "solvants polyfluorés volatils" s'est avérée permettre d'obtenir un séchage particulièrement rapide après application de la composition sur la peau, ce qui n'est pas le cas des compositions de l'art antérieur.

Un autre avantage particulièrement appréciable est de pouvoir obtenir les compositions en parfaite sécurité, dans la mesure où les solvants polyfluorés volatils ne possèdent pas de point éclair, ce qui permet de réaliser des compositions à tempé-rature élevée, et présente donc un avantage certain lorsque l'on utilise des substances ayant un point de fusion supérieur à la température ambiante.

Enfin, l'utilisation de solvants polyfluorés volatils facilite l'incorporation dans les compositions de concentrations élevées en dérivés fluorés non-volatils, ceux-ci étant totalement miscibles avec les solvants polyfluorés volatils. On peut ainsi obtenir un film résiduel hautement fluoré, brillant ou satiné, et résistant à l'eau et aux corps gras.

La présente invention a donc pour objet, l'utilisation dans une composition cosmétique de soin des ongles, d'au moins un solvant polyfluoré volatil, en tant qu'agent accélérateur de séchage, ledit solvant ayant une pression de vapeur supérieure à 20 mba (2000 Pa) à 25°C, de préférence supérieure à 40 mba (4000 Pa) et étant un composé de formule :

CH₃ - (CH₂)ₙ - [Z]ₜ - X - CF₃ (I)

dans laquelle :
t est 0 ou 1,
X est un radical perfluoroalkyle divalent, linéaire ou ramifié, ayant de 2 à 5 atomes de carbone, et
Z représente O, S ou NR, R étant hydrogène, un radical - (CH₂)ₙ - CH₃ ou - (CF₂)ₘ - CF₃ n est 0, 1, 2 ou 3 et m étant 2, 3, 4 ou 5.

Parmi les composés fluoroalkyles ou hétérofluoroalkyles de formule (I) on peut notamment citer le méthoxynonafluorobutane vendu sous la dénomination de HFE-7100 par la société 3M ou l'éthoxynonafluorobutane vendu sous la dénomination de « HFE-7200® » par la société 3M.

Les solvants polyfluorés volatils de formule (I) ci-dessus doivent également satisfaire au critère du point d'ébullition, celui-ci devant être compris entre 20 et 75°C et de préférence entre 25 et 65°C.

Dans les compositions selon l'invention, la proportion en solvant polyfluoré volatil est généralement comprise entre 2 et 98 % en poids, mais de préférence entre 5 et 70 % en poids par rapport au poids total de la composition.

Selon un mode de réalisation préféré de l'invention les compositions contiennent des particules de pigment et/ou de colorant et/ou de charge.

Les pigments des compositions selon l'invention ainsi que les colorants et charges, se présentent sous forme de particules très fines ayant une taille moyenne de particules comprise entre environ 0,02 à 50 µm.

Les pigments des compositions peuvent être minéraux ou organiques ou encore sous forme de laques métalliques. Parmi ces pigments on peut citer le dioxyde de titane, l'oxyde de zinc, le D&C Red No. 36 et le D&C Orange No. 17, les laques de calcium de D&C Red No. 7, 11, 31 et 34, la laque de baryum de D&C Red No. 12, la laque de strontium D&C Red No. 13, les laques d'aluminium de FD&C Yellow No. 5, de FD&C Yellow No. 6, de D&C Red No. 27, de D&C Red No. 21, de FD&C Blue No. 1, les oxydes de fer, le violet de manganèse, l'oxyde de chrome et le bleu d'outremer.

Les charges peuvent être d'origine naturelle ou synthétique. Parmi celles-ci, on peut notamment citer:
a) les poudres minérales telles que le talc, le kaolin, le mica, la silice, les silicates, l'alumine, les zéolites, l'hydroxyapatite, la séricite, les micatitanes, le sulfate de baryum, l'oxychlorure de bismuth, le nitrure de bore et les poudres métalliques telles que la poudre d'aluminium ;
b) les poudres végétales telles que les poudres d'amidon, de maïs, de froment ou de riz ;
c) les poudres organiques telles que les poudres de nylon, de polyamide, de polyester, de polytétrafluoroéthylène ou de polyéthylène.

Ces différentes poudres peuvent en outre être enrobées, par exemple, par des sels métalliques d'acides gras, des acides aminés, de la lécithine, du collagène, des composés siliconés, des composés fluorés ou par tout autre enrobage usuel.

Parmi les colorants on peut citer les dérivés d'éosine tel que le D&C Red No. 21 et les dérivés de fluoroscéine halogéné tel que le D&C Red No. 27, le D&C Orange No. 5 en association avec le D&C Red No. 21 et le D&C Orange No. 10.

Selon les compositions, les colorants peuvent se présenter soit sous forme de particules soit sous forme solubilisée dans le véhicule de la composition.

Dans les compositions selon l'invention, la proportion en au moins un pigment et/ou colorant est généralement comprise entre environ 0,1 et 15 % en poids par rapport au poids total de la composition.

Les charges peuvent être généralement présentes en une proportion maximum d'environ 98 % en poids par rapport au poids total de la composition.

Selon un premier mode de réalisation particulier des compositions selon l'invention, celles-ci sont anhydres et comprennent une phase grasse en une proportion comprise entre environ 0,3 à 90 % en poids par rapport au poids total de la composition.

La phase grasse est généralement constituée d'un ou plusieurs corps gras qui peuvent être choisis parmi les huiles, les cires, les gommes et/ou les corps gras dits pâteux.

### A-Les huiles de la phase grasse peuvent être d'origine minérale, animale, végétale ou de synthèse, celles-ci pouvant être volatiles ou non à température ambiante.

Comme huile d'origine minérale, on peut citer notamment l'huile de paraffine et l'huile de vaseline.

Comme huile d'origine animale, on peut citer notamment le squalane ou perhydrosqualène.

Comme huile d'origine végétale, on peut citer notamment l'huile d'amande douce, l'huile de calophyllum, l'huile de palme, l'huile d'avocat, l'huile de jojoba, l'huile de sésame, l'huile d'olive, l'huile de ricin et les huiles de germes de céréales telles que par exemple l'huile de germes de blé.

Comme huile de synthèse, on peut citer notamment :
(1) les esters de formule suivante :

   R₁ - COOR₂

   dans laquelle :
   R₁ représente le reste d'un acide gras supérieur ayant de 7 à 20 atomes de carbone et
   R₂ représente un radical hydrocarboné ayant de 3 à 30 atomes de carbone.

   Parmi ces esters, on peut notamment citer : l'huile de Purcellin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate de décyle, le laurate d'hexyle, l'isononanoate d'isononyle, les esters dérivés de l'acide lanolique tels que le lanolate d'isopropyle et le lanolate d'isocétyle.
   Comme autres huiles de synthèse, on peut citer en outre les isoparaffines, l'isododécane, l'isohexadécane, les polyisobutènes et le polyisobutène hydrogéné ainsi que les acétylglycérides, les octanoates et décanoates de polyalcools et de polyalcools tels que ceux de glycol et de glycérol, les ricinoléates d'alcools et de polyalcools tels que celui d'alcool cétylique, le dicaprylate de propylène glycol et d'adipate de diisopropyle ;
(2) les alcools gras tels que l'alcool oléique, l'alcool linoléique, l'alcool linolénique, l'alcool isostéarylique et l'octyldodécanol ;
(3) les huiles de silicone telles que les polydiorganosiloxanes linéaires éventuellement fonctionnalisées, les polydiorganosiloxanes cycliques et en particulier les cyclotétra- et penta-diméthicones et les organopolysiloxanes tels que les alkyl, alcoxy ou phényl diméthicones et en particulier la phényltriméthicone ;
(4) les huiles fluorées non volatiles telles que la perfluorodécaline, le perfluorophénanthrène, les perfluoroalcanes et les perfluoropolyéthers et les huiles hydrocarbonées partiellement fluorées.

Selon une forme particulièrement préférée de l'invention, on utilise des perfluoropolyéthers non volatils répondant aux formules suivantes :
1) 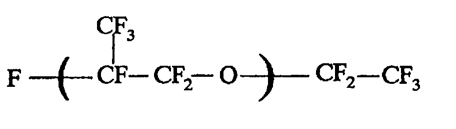 dans laquelle :
   n = 7 à 30,
2) 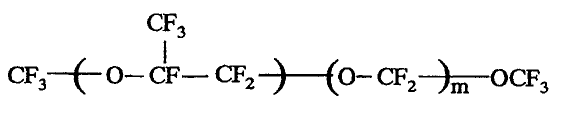 dans laquelle :
   n et m = 20 à 40.

Parmi ceux-ci, on peut citer ceux vendus sous les dénominations de « FOMBLIN C® » et de « GALDEN® » par la société AUSSIMONT, ou encore sous la dénomination « FLUORTRESS LM 36® » par la société DU PONT.

### B - Les cires de la phase grasse peuvent être d'origine minérale, fossile, animale, végétale, de synthèse ou bien encore être des huiles hydrogénées ou des esters gras concrets à 25°C.

Parmi les cires minérales, on peut citer notamment les cires microcristallines, la paraffine, la vaseline et la cérésine.

Parmi les cires fossiles, on peut citer l'ozokérite et la cire de montan.

Parmi les cires d'origine animale, on peut citer la cire d'abeilles, le spermaceti, la cire de lanoline ainsi que les dérivés issus de la lanoline tels que les alcools de lanoline, la lanoline hydrogénée, la lanoline hydroxylée, la lanoline acétylée, les acides gras de lanoline et l'alcool de lanoline acétylé.

Parmi les cires d'origine végétale, on peut citer notamment la cire de candellila, la cire de carnauba, la cire du Japon et le beurre de cacao.

Parmi les cires de synthèse, on peut citer notamment les homopolymères d'éthylène et les copolymères d'éthylène et d'un monomère répondant à la formule suivante :

CH₂ = CH - R₃

dans laquelle :
R₃ représente un radical alkyle ayant de 1 à 30 atomes de carbone, un radical aryle ou aralkyle.

Le radical alkyle ayant de 1 à 30 atomes de carbone est de préférence le radical méthyle, éthyle, propyle, isopropyle, butyle, décyle, dodécyle ou octadécyle.

On peut également utiliser les cires obtenues par synthèse Fisher-Tropsch ainsi que les cires de silicone.

Parmi les huiles hydrogénées concrètes à 25°C, on peut citer notamment l'huile de ricin hydrogénée, l'huile de palme hydrogénée, le suif hydrogéné et l'huile de coco hydrogénée.

Parmi les esters gras concrets à 25°C, on peut citer notamment le mono-myristate de propylène glycol et le myristate de myristyle.

Comme cires utilisables dans les compositions selon l'invention, on peut encore citer l'alcool cétylique, l'alcool stéarylique, les mono-, di- et triglycérides concrets à 25°C, le monoéthanolamide stéarique, la colophane et ses dérivés tels que les abiétates de glycol et de glycérol, les sucro-glycérides et les oléates, myristates, lanolates, stéarates et dihydroxystéarates de calcium, de magnésium, de zinc et d'aluminium.

### C - Les corps gras de type pâteux peuvent être d'origine minérale, animale, végétale ou de synthèse.

Parmi les corps gras pâteux, on peut citer notamment les esters de synthèse tels que le propionate d'arachidyle, le polylaurate de vinyle, les cires de polyéthylène et les organopolysiloxanes tels que les alkyldiméthicones, les alcoxydiméthicones ou les esters diméthicones.

Les compositions anhydres telles que définies ci-dessus, peuvent bien entendu contenir en outre un ou plusieurs additifs ou adjuvants cosmétiques ou dermatologiques conventionnels.

Ces compositions anhydres se présentent sous forme de produits huileux fluides pour le soin des ongles, la phase grasse contenant au moins un agent actif.

Parmi les agents actifs de ces compositions pour le soin des ongles on peut notamment mentionner les céramides, le phytantriol, le D-panthénol, les α-hydroxyacides, les filtres UV, les vitamines, la kératine et la biotine.

Les compositions telles que décrites ci-dessus, qu'elles soient du type anhydre ou sous forme d'une dispersion, présentent d'excellentes propriétés cosmétiques telles que notamment une excellente facilité d'application, une grande douceur et conduisent à l'obtention d'un maquillage homogène.

Les compositions telles qu'elles viennent d'être décrites ci-dessus, peuvent en outre contenir un ou plusieurs adjuvants cosmétiques conventionnels tels que des vitamines, des hormones, des agents antioxydants, des agents rhéologiques, des conservateurs, des parfums, des épaississants, des agents hydratants, des agents humectants, des polymères anioniques, non-ioniques ou amphotères, ou des actifs cosmétiques ou dermatologiques.

L'invention sera maintenant illustrée par l'exemple suivant, dont les quantités sont exprimées en poids.

### Exemple : Produit pour le soin des ongles

On prépare un produit huileux pour le soin des ongles en procédant au mélange des ingrédients suivants :
- Méthoxynonafluorobutane 10,0 g
- Acétobutyrate de cellulose 0,5 g
- Alcool isopropylique 5,0 g
- Monométhyléther de propylène glycol 3,0 g
- Huile de silicone volatile 10,0 g
- Huile végétale 64,5 g
- Substance active (D-panthénol) 1,0 g
- Colorants
- Silice pyrogénée 1,0 g
- Huile minérale q.s.p. 100,0 g

Ce produit pour le soin des ongles s'applique facilement et pénètre par massage dans la matrice de l'ongle et dans la tablette unguéale ; sa vitesse de séchage est très rapide.

Dans cet exemple, le méthoxynonafluorobutane peut être avantageusement remplacé par une quantité équivalente d'éthoxynonafluorobutane.

## Revendications

1. Utilisation dans une composition cosmétique de soin des ongles d'au moins un solvant polyfluoré volatil, en tant qu'agent accélérateur de séchage, ledit solvant ayant une pression de vapeur supérieure à 20 mba (2000 Pa) à 25°C et étant un composé de formule :
CH₃-(CH₂)ₙ-[Z]ₜ-X-CF₃ (I)
dans laquelle :
n est 0, 1, 2 ou 3
t est 0 ou 1,
X est un radical divalent perfluoroalkyle, linéaire ou ramifié, ayant de 2 à 5 atomes de carbone, et
Z représente O, S ou NR, R étant hydrogène, un radical -(CH₂)ₙ-CH₃ ou -(CF₂)ₘ-CF₃, et m étant 2, 3, 4 ou 5.

2. Utilisation selon la revendication 1, **caractérisée par le fait que** le composé accélérateur de séchage répond à la formule :
CH₃-(CH₂)ₙ-[Z]ₜ-X-CF₃
dans laquelle :
t est 1,
n est 0, 1, 2 ou 3,
X est un radical perfluoroalkyle divalent, linéaire ou ramifié, ayant 2 à 5 atomes de carbone et Z représente O.

3. Utilisation selon la revendication 1 ou 2, **caractérisée par le fait que** le composé accélérateur de séchage est le méthoxynonafluorobutane ou l'éthoxynonafluorobutane.

4. Utilisation selon l'une quelconque des revendications précédentes, **caractérisée par le fait que** le composé accélérateur de séchage est présent dans la composition en une proportion comprise entre 2 et 98 % en poids par rapport au poids total de la composition.

5. Utilisation selon l'une quelconque des revendications précédentes, **caractérisée par le fait que** la composition contient au moins un agent actif choisi parmi les céramides, le phytantriol, le D-panthénol, les α -hydroxyacides, les filtres UV, les vitamines, la kératine et la biotine.

6. Utilisation selon l'une quelconque des revendications précédentes, **caractérisée par le fait que** la composition contient au moins un pigment et/ou colorant et/ou charge sous forme de particules.

7. Utilisation selon la revendication 6, **caractérisée par le fait que** la proportion en pigment et/ou colorant dans la composition est comprise entre 0,1 et 15 % en poids par rapport au poids total de la composition.

8. Utilisation selon la revendication 6, **caractérisée par le fait que** la proportion en charge dans la composition est au maximum de 98 % en poids par rapport au poids total de la composition.

## Patentansprüche

1. Anwendung von mindestens einem flüchtigen, polyfluoriertem Lösungsmittel als Beschleunigungsmittel für den Trocknungsvorgang in einem kosmetischen Pflegeprodukt für die Fingernägel, wobei das Lösungsmittel einen Dampfdruck von mehr als 20 mbar (2000 Pa) bei 25 °C aufweist und eine Zusammensetzung mit der folgenden Formel ist:
CH₃-(CH₂)ₙ-[Z]ₜ-X-CF₃ (I)
wobei:
n = 0, 1, 2 oder 3
t = 0 oder 1
X ein lineares oder verzweigtes zweiwertiges Perfluoroalkyl mit 2 bis 5 Kohlenstoffatome ist und
Z: O, S oder NR darstellt, wobei R Wasserstoff ist, ferner ein -(CH₂)ₙ-CH₃oder ein -(CF₂)ₘ-CF₃ Radikal, wobei m = 2, 3, 4 oder 5.

2. Verwendung nach Anspruch 1, **dadurch gekennzeichnet, dass** die trocknungsbeschleunigende Komponente die folgende Formel hat:
CH₃-(CH₂)ₙ-[Z]ₜ-X-CF₃,
wobei:
t = 1
n = 0, 1, 2 oder 3,
X ein lineares oder verzweigtes zweiwertiges Perfluoroalkyl mit 2 bis 5 Kohlenstoffatome ist und wobei Z die Darstellung von O ist.

3. Verwendung nach Anspruch 1 oder 2, **dadurch gekennzeichnet, dass** es sich bei der Zusammensetzung zum Beschleunigen des Trockenvorgangs um Methoxynonafluorobutan oder um Ethoxynonafluorobutan handelt.

4. Verwendung nach einem der vorhergehenden Ansprüche, **dadurch gekennzeichnet, dass** die Zusammensetzung zum Beschleunigen des Trockenvorgangs im Produkt vorhanden ist, wobei sein Anteil zwischen 2 und 98 Gewichtsprozent im Verhältnis zum Gesamtgewicht der Zusammensetzung beträgt.

5. Verwendung nach einem der vorhergehenden Ansprüche, **dadurch gekennzeichnet, dass** die Zusammensetzung mindestens einen aktiven Agenten umfasst, der unter den Zeramiden, dem Phytantriol, dem D-Panthenol, den α-Hydroxylsäuren, den UV-Filtern, den Vitaminen, dem Keratin und dem Biotin gewählt ist.

6. Verwendung nach einem der vorhergehenden Ansprüche, **dadurch gekennzeichnet, dass** die Zusammensetzung mindestens ein Pigment und/oder Farbstoff und/oder teilchenförmige Ladung enthält.

7. Verwendung nach Anspruch 6, **dadurch gekennzeichnet, dass** der Pigmentund/oder Farbstoffgehalt in der Zusammensetzung zwischen 0,1 und 15 Gewichtsprozent im Verhältnis zum Gesamtgewicht der Zusammensetzung liegt.

8. Verwendung nach Anspruch 6, **dadurch gekennzeichnet, dass** der Ladungsinhalt der Zusammensetzung höchstens 98 Gewichtsprozent im Verhältnis zum Gesamtgewicht der Zusammensetzung liegt.

## Claims

1. Use in a nail care cosmetic composition of at least one volatile polyfluorinated solvent as a drying accelerator, the said solvent having a vapour pressure above 20 mbar (2000 Pa) at 25°C and being a compound of formula:
CH₃-(CH₂)ₙ-[Z]ₜ-X-CF₃ (1)
in which:
n is 0, 1, 2 or 3
t is 0 or 1,
X is a linear or branched divalent perfluoroalkyl radical having 2 to 5 carbon atoms, and
Z represents O, S or NR, R being hydrogen, a - (CH₂)ₙ-CH₃ or (CF₂)ₘ-CF₃ radical, and m being 2, 3, 4 or 5.

2. Use according to claim 1, **characterized in that** the accelerated drying compound corresponds to the formula:
CH₃-(CH₂)ₙ-[Z]ₜ-X-CF₃
in which:
t is 1,
n is 0, 1, 2 or 3
X is a linear or branched divalent perfluoroalkyl radical having 2 to 5 carbon atoms, and
Z represents 0.

3. Use according to claim 1 or 2, **characterized in that** the accelerated drying compound is methoxynonafluorobutane or ethoxynonafluorobutane.

4. Use according to any one of the preceding claims, **characterized in that** the accelerated drying compound is present in the composition in a proportion of between 2 and 98 % by weight based on the total weight of the composition.

5. Use according to any one of the preceding claims, **characterized in that** the composition contains at least one active agent chosen from ceramides, phytantriol, D-panthenol, _-hydroxyacids, UV filters, vitamins, keratin and biotin.

6. Use according to any one of the preceding claims, **characterized in that** the composition contains at least one pigment and/or colouring agent and/or filler in the form of particles.

7. Use according to claim 6 **characterized in that** the proportion of pigment and/or colouring agent in the composition lies between 0.1 and 15 % by weight based on the total weight of the composition.

8. Use according to claim 6 **characterized in that** the proportion of filler in the composition is a maximum of 98 % by weight based on the total weight of the composition.
